# EUROPEAN PATENT APPLICATION

(11) **EP 3 275 471 A1**
(43) Date of publication of application: **31.01.2018**
(21) Application number: 16769138.5
(22) Date of filing: 25.03.2016
(51) Int. Cl.: A61L 27/36, C12N 5/077, C07K 14/475

(54) **THREE-DIMENSIONAL STRUCTURE FOR CARDIAC MUSCULAR TISSUE REGENERATION AND MANUFACTURING METHOD THEREFOR**

(30) Priority: 26.03.2015 KR 20150042418
(71) Applicant: T&R Biofab Co., Ltd., Siheung-si, Gyeonggi-do 15073 (KR)
(72) Inventor: CHO, Dong Woo, Seoul 05065 (KR); JANG, Jinah, Pohang-si Gyeongsangbuk-do 37673 (KR); PARK, Hun Jun, Seoul 06277 (KR)
(74) Representative: Hoppe, Georg Johannes
(86) International application number: PCT/KR2016/003079
(87) International publication number: WO 2016/153320

(57) **Abstract**

The present invention provides a preparation method of a three-dimensional construct for regenerating a cardiac muscle tissue comprising: a step of forming a three-dimensional construct by printing and crosslinking the first bioprinting composition comprising a tissue engineering construct forming solution containing decellularized extracellular matrix and a crosslinking agent, and cardiac progenitor cells, and the second bioprinting composition comprising the tissue engineering construct forming solution, mesenchymal stem cells and a vascular endothelial growth factor, to arrange the first bioprint layer and the second bioprint layer alternately; and a step of obtaining a crosslink-gelated three-dimensional construct by thermally gelating the crosslinked three-dimensional construct, and a three-dimensional construct for regenerating a cardiac muscle tissue, and the preparation method according to the present invention not only equally positions the cardiac progenitor cells in the construct but also implements a vascular network composed of vascular cells in the construct, so that the viability of cells can be maintained for a long time and the cell transfer efficiency into the myocardium can be significantly improved.

## Description

### [Technical Field]

The present invention relates to a three-dimensional construct for regenerating a cardiac muscle tissue and a method for preparing thereof. More specifically, the present invention relates to a preparation method of a three-dimensional construct for regenerating a cardiac muscle tissue comprising; a step of forming a three-dimensional construct by printing and crosslinking the first bioprinting composition comprising a tissue engineering construct forming solution containing decellularized extracellular matrix and a crosslinking agent, and cardiac progenitor cells, and the second bioprinting composition comprising the tissue engineering construct forming solution, mesenchymal stem cells and a vascular endothelial growth factor, to arrange the first bioprint layer and the second bioprint layer alternately; and a step of obtaining a crosslink-gelated three-dimensional construct by thermally gelating the crosslinked three-dimensional construct, and a three-dimensional construct for regenerating a cardiac muscle tissue.

### [Background Art]

A three-dimensional printing means constructing complex skeletal structure through a layer-by-layer process, after converting arbitrary shape information obtained from medical data of tissues or organs with complicated shapes into G-cods. This three-dimensional printing is also referred to 'three-dimensional bioprinting (3D bioprinting). For example, 'Multi-headtissue and organ printing system' is one of the representative three-dimensional printing technologies, and it is composed of two pneumatic syringes which spray materials pneumatically and two piston-type syringes which precisely spray in units of nanoliter using a step motor, and various materials can be used at the same time.

Typically, a construct is prepared by attaching a thermoplastic biocompatible polymer such as PLA (Polylactic Acid), PGA (Poly-glycolic Acid), PLGA (Poly-lactic-co-glycolid Acid), PCL (Polycaprolactone), or a mixture thereof to pneumatic syringes. In addition, a three-dimensional construct is prepared using piston-type syringes having a hydrogel consisting of collagen, hyaluronic acid, gelatin, alginate, chitosan or fibrin, etc.

As the bioprinting requires distribution of cell-containing media, an important aspect of bioprinting is that the printing process must be cytocompatible. This limitation reduces the choice of materials, due to the need to perform in an aqueous or aqueous gel environment. Thus, a hydrogel using materials such as gelatin, gelatin/chitosan, gelatin/alginate, gelatin/fibronectin, Lutrol F127/alginate, and alginate, etc. has been used for bioprinting for preparing various tissues from liver to bone. The hydrogel or a mixture of the hydrogel and cells, etc. used for bioprinting is also referred to 'bioink'.

In general, cells remain specifically located at their original location during entire incubation period, since the cells cannot sustain or degrade the surrounding alginate gel matrix (Fedorovich, N. E. et al. Tissue Eng. 13, 1905-1925 (2007)). Thus, although there are some success reports on bioprinting of cell-print constructs, minimal cell-material interactions and inferior tissue formation are the most important problems. Indeed, these materials cannot exhibit complexity of natural extracellular matrices (ECMs), and thus they are not sufficient to reproduce the microenvironment having typical cell-cell connections and three-dimensional (3D) cell configurations of biological tissues.

Thus, cells in the hydrogel cannot exhibit unique morphology and function of living tissues in vivo. Therefore, it would be ideal, if a natural microenvironment similar to the parent tissue of the cells is provided to the cells. A decellularized extracellular matrix (dECM) is the best choice for this, because any natural or artificial material cannot reproduce all of the natural extracelluar matrix properties.

Moreover, the ECM of each tissue is unique in terms of composition and topology, and the composition and anatomy characteristics are generated through dynamic and interactive interactions between resident cells and the microenvironment. Recent studies of cells and ECM isolated from tissues and organs emphasize the need for tissue specificity to preserve selected cell functions and phenotypes (Sellaro, T. L. et al. Tissue Eng. Part A 16, 1075-1082 (2010); Petersen, T. H. et al. Science 329, 538-541 (2010); Uygun, B. E. et al. Nat. Med. 16, 814-821 (2010); Ott, H. C. et al. Nat. Med. 16, 927-933 (2010); Flynn, L. E. Biomaterials 31, 4715-4724 (2010)).

The collected dECM materials are typically processed as a tow-dimensional (2D) scaffold from various tissues including skin, small intestinal submucosa, and in the early stage, permeable or seeded cells depend on diffusion of oxygen and nutrients for survival until a supporting vascular network is established.

However, printed tissue analogue structure requires a preparation method that devises an open porous 3D structure to allow flow of nutrients. The present inventors have developed a three-dimensional printing method for printing of cell-laden structures using dECM bioink which can provide optimal microenvironment appropriate to growth of 3D structure tissue, and the cell-laden structure can reproduce unique cell morphology and function (Falguni Pati, et al., Nat Commun. 5,3935 (2014)).

On the other hand, ischemic heart disease has a very high prevalence worldwide and is the number one cause of death in the whole single disease. Korea is also experiencing rapid increases in ischemic heart disease patients due to socioeconomic development and westernized lifestyle. Furthermore, in severe end-stage heart failure patient, there is no cure method other than cardiac transplantation or mechanical left ventricular assist device. However, there are problems such as shortage of donor organs, difficulty in securing organs, high mortality rate and expensive treatment cost, etc.

It is expected that the treatment using stem cell multipotency will fundamentally treat the regeneration of damaged tissues. In recent years, various studies on increasing efficiency of heart disease treatment by mixing adult stem cells with vascular endothelial cells and other stromal cells or various hydrogels and injecting them, thereby promoting differentiation into myocardial cells and preventing cells from separating from lesions. In addition, studies on stimulating biological environment of stem cells and maximizing interactions among cells by preparing cell sheets using stem cells or stem cells and various adult cells have been actively carried out.

### [Disclosure]

### [Technical Problem]

One embodiment of the present invention relates to a preparation method of a three-dimensional construct for regenerating a cardiac muscle tissue comprising; a step of forming a three-dimensional construct by printing and crosslinking the first bioprinting composition comprising a tissue engineering construct forming solution containing decellularized extracellular matrix and a crosslinking agent, and cardiac progenitor cells, and the second bioprinting composition comprising the tissue engineering construct forming solution, mesenchymal stem cells and a vascular endothelial growth factor, to arrange the first bioprint layer and the second bioprint layer alternately; and a step of obtaining a crosslink-gelated three-dimensional construct by thermally gelating the crosslinked three-dimensional construct.

Another embodiment of the present invention relates to a three-dimensional construct for regenerating a cardiac muscle tissue formed by crosslinking and thermally gelating the first bioprint layer and the second bioprint layer which are prepared by printing the first bioprinting composition and the second bioprinting composition.

### [Technical Solution]

The present inventors have conducted various studies to develop a method for preparing a three-dimensional construct in which microenvironment of cardiac muscle tissue is realized. Accordingly, the present inventors have found that a microenvironment of the cardiac muscle tissue can be effectively implemented, when a three-dimensional printing is performed through layer-by-layer process using a bioprinting composition comprising decellularized extracellular matrix and cardiac progenitor cells and a bioprinting composition comprising decellularized extracellular matrix, mesenchymal stem cells and vascular endothelial growth factor (VEGF) alternately. In particular, it has been found that this three-dimensional printing method not only equally positions cardiac progenitor cells in the construct but also implement vascular network consisting of vascular cells in the construct, thereby maintaining the cell viability for a long time and remarkably enhancing cell transfer efficacy into the myocardium.

Accordingly, one example of the present invention,
relates to a preparation method of a three-dimensional construct for regenerating a cardiac muscle tissue comprising,
(a) a step of forming a three-dimensional construct by printing and crosslinking the first bioprinting composition comprising a tissue engineering construct forming solution containing decellularized extracellular matrix and a crosslinking agent, and cardiac progenitor cells, and the second bioprinting composition comprising the tissue engineering construct forming solution, mesenchymal stem cells and a vascular endothelial growth factor, to arrange the first bioprint layer and the second bioprint layer alternately; and
(b) a step of obtaining a crosslink-gelated three-dimensional construct by thermally gelating the crosslinked three-dimensional construct.

As above, the three-dimensional construct prepared by performing layer-by-layer process through printing using the first bioprinting composition comprising decellularized extracellular matrix and cardiac progenitor cells; and the second bioprinting composition comprising decellularized extracellular matrix, mesenchymal stem cells and vascular endothelial growth factor alternately, has an effect of remarkably enhancing cell transfer efficacy into the myocardium, since microenvironment of cardiac muscle tissue can be effectively implemented and the cell viability can be maintained for a long time by not only equally positioning cardiac progenitor cells in the construct but also implementing vascular network consisting of vascular cells in the construct.

The decellularized extracellular matrix may be derived from a cardiac tissue. Specifically, it may be obtained by decellularizing cardiac tissues excreted in vitro, and may be obtained by decellularizing tissues released from mammals such as human, pig, cow, rabbit, dog, goat, sheep, chicken, horse, etc., and it may be obtained by decellularizing preferably tissues obtained from pig, more preferably cardiac tissues obtained from pig, but not limited thereto.

The decellularization may be carried out by using or slightly modifying a known method, for example, a method disclosed in Ott, H. C. et al. Nat. Med. 14, 213-221 (2008), Yang, Z. et al. Tissue Eng. Part C Methods 16, 865-876 (2010), or the like. Preferably, it may be performed using the decellularizing method which the present inventors have reported, in that disclosed in Falguni Pati, et al., Nat Commun. 5, 3935 (2014). The decellularized extracellular matrix may be typically stored in the form of a lyophilized powder.

The amount of the decellularized extracellular matrix used is not particularly limited, but for example, it may be used in an amount of 1 to 4 % by weight, 1 to 3 % by weight, 2 to 4 % by weight, preferably 2 to 3 % by weight, based on the total weight of composition. For example, the decellularized extracellular matrix of the first bioprinting composition may be contained in an amount of 1 to 4 % by weight based on the total weight of the first bioprinting composition; and the decellularized extracellular matrix of the second bioprinting composition may be contained in an amount of 1 to 4 % by weight based on the total weight of the second bioprinting composition. If the content is low, out of the above range, there is a problem that materials may not be laminated, and if the content is high, there is a problem that cells encapsulated therein may be killed.

The cardiac progenitor cells and vascular endothelial cells are derived from animals including human and commercially available, and they may be cultured and propagated by known methods.

The cardiac progenitor cells may be contained in a range of 10⁵ to 10⁸ cells/ml, 10⁶ to 10⁸ cells/ml, 10⁵ to 5 X 10⁷ cells/ml, preferably 10⁶ to 5 X 10⁷ cells/ml, in the first bioprinting composition. If the content is low, out of the above range, there is a problem that their efficacy is low, and if the content is high, there is a problem that the three-dimensional shape is not maintained, because the decellularized extracellular matrix cannot be crosslinked, even though bioink-decellularized extracellular matrix that covers cells is crosslinked.

The mesenchymal stem cells may be contained in a range of 10⁵ to 10⁸ cells/ml, 10⁶ to 10⁸ cells/ml, 10⁵ to 5 X 10⁷ cells/ml, preferably 10⁶ to 5 X 10⁷ cells/ml, in the second bioprinting composition. If the content is low, out of the above range, there is a problem that their efficacy is low, and if the content is high, there is a problem that the three-dimensional shape is not maintained, because the decellularized extracellular matrix cannot be crosslinked, even though bioink-decellularized extracellular matrix that covers cells is crosslinked.

In addition, the vascular endothelial growth factor may be contained in a range of 50 to 1000ng/ml, 50 to 900ng/ml, 50 to 800ng/ml, 50 to 700ng/ml, 50 to 600ng/ml, 50 to 500ng/ml, 50 to 400ng/ml, 50 to 300ng/ml, 50 to 200ng/ml, 100 to 1000ng/ml, 100 to 900ng/ml, 100 to 800ng/ml, 100 to 700ng/ml, 100 to 600ng/ml, 100 to 500ng/ml, 100 to 400ng/ml, 100 to 300ng/ml, preferably 100 to 200 ng/ml, in the second bioprinting composition. If the content is low, out of the above range, there is a problem that their efficacy is low, and if the content is high, there is a problem due to excessive angiogenesis.

It is preferable that the first bioprinting composition and the second bioprinting composition has a form of viscoelastic homogeneous solution having a pH in a range of 6.5 to 7.5.

Thus, the tissue engineering construct forming solution may further comprise an acid; a protease; and a pH control agent. The acid, protease, and pH control agent may be comprised in an aqueous medium, but not limited thereto.

In addition, the acid may be one or more kinds of acids selected from the group consisting of acetic acid and hydrochloric acid, but not limited thereto. The acid conducts a function of dissolving the decellularized extracellular matrix, and preferably acetic acid, hydrochloric acid, etc. may be used, and more preferably may be used as a form of 0.01 to 10 M, 0.01 to 9 M, 0.01 to 8 M, 0.01 to 7 M, 0.01 to 6 M, 0.01 to 5 M, 0.01 to 4 M, 0.01 to 3 M, 0.01 to 2 M, 0.01 to 1 M, 0.1 to 10 M, 0.1 to 9 M, 0.1 to 8 M, 0.1 to 7 M, 0.1 to 6 M, 0.1 to 5 M, 0.1 to 4 M, 0.1 to 3 M, 0.1 to 2 M, 0.1 to 1 M acetic acid aqueous solution, for example, approximately 0.5M acetic acid aqueous solution, or 0.01 to 10 M, 0.01 to 9 M, 0.01 to 8 M, 0.01 to 7 M, 0.01 to 6 M, 0.01 to 5 M, 0.01 to 4 M, 0.01 to 3 M, 0.01 to 2 M, 0.01 to 1 M, 0.01 to 0.1 M hydrochloric acid aqueous solution, for example, approximately 0.01M hydrochloric acid aqueous solution.

The protease conducts digestion function of telopeptides of the decellularized extracellular matrix, and it may be one or more kinds of selected from the group consisting of pepsin and matrix metalloproteinase, but not limited thereto. The amount of the protease used differs depending on the content of decellularized extracellular matrix, but it may be used as a ratio of 5 to 30 mg, 5 to 25 mg, 10 to 30 mg, preferably 10 to 25 mg based on 100 mg of decellularized extracellular matrix.

The pH control agent is for controlling the pH of composition as pH 6.5 to 7.5, pH 6.6 to 7.4, pH 6.7 to 7.3, pH 6.8 to 7.2, pH 6.9 to 7.1, preferably pH 7 by neutralizing the acid used for dissolution of the decellularized extracellular matrix, and for example, it may be sodium hydroxide, but not limited thereto.

In one embodiment, the first bioprinting composition and the second bioprinting composition, independently comprise, based on the total weight of each composition, 0.03 to 30 % by weight of one or more kinds of acids selected from the group consisting of acetic acid and hydrochloric acid; 0.1 to 0.4 % by weight of one or more kinds of proteases selected from the group consisting of pepsin and matrix metalloproteinase; and a pH control agent additionally.

The first bioprinting composition and the second bioprinting composition, respectively independently comprise one or more kinds selected from the group consisting of cell, growth factor and enzyme additionally.

The cell may be one or more kinds selected from the group consisting of endothelial progenitor cells, endothelial cells and cardiomyocytes.

The growth factor may be one or more kinds selected from the group consisting of fibroblast growth factor (FGF), platelet-derived growth factor (PDGF), angiopoietin-1, transforming growth factor beta (TGF-β), erythropoietin (EPO), stem cell factor (SCF), epidermal growth factor (EGF) and colony stimulating factor (CSF).

The enzyme may be one or more kinds selected from the group consisting of matrix metalloproteinase (MMP) and tissue inhibitor of matrix metalloproteinase (TIMP).

In one example, the first bioprinting composition and the second bioprinting composition, respectively independently,
may comprise additionally;
one or more kinds of selected from the cell group consisting of endothelial progenitor cells, endothelial cells and cardiomyocytes; and
one or more kinds of growth factors selected from the growth factor group consisting of fibroblast growth factor (FGF), platelet-derived growth factor (PDGF), angiopoietin-1, transforming growth factor beta (TGF-β), erythropoietin (EPO), stem cell factor (SCF), epidermal growth factor (EGF) and colony stimulating factor (CSF).

In addition, the first bioprinting composition and the second bioprinting composition further comprising the one or more kinds of cells and one or more kinds of growth factors, respectively independently,
may further comprise additionally;
one or more kinds of enzymes selected from the enzyme group consisting of matrix metalloproteinase (MMP) and tissue inhibitor matrix metalloproteinase (TIMP).

The growth factor may be comprised as a form of endothelial cell growth supplement (ECGS), but not limited thereto.

It is preferable that the first bioprinting composition and the second bioprinting composition is a viscoelastic material in which the viscosity lowers as the shear rate increases, and for example, it is preferable the viscosity at a shear rate of 1 s⁻¹ measured at approximately 15°C in the range of 1 to 30 Pa·S. The viscosity may be controlled by properly controlling the amount of an aqueous medium (for example, water, distilled water, PBS, physiological saline solution, etc.).

The printing using the first bioprinting composition and the second bioprinting composition alternately may be carried out using a known three-dimensional printing method (for example, a printing method using 'Multi-headtissue and organ printing system'), according to a method disclosed, Falguni Pati, et al., Nat Commun. 5, 3935 (2014), etc. and for example, the printing may be conducted using two syringes of multi-headtissue and organ printing system.

That is, a polycaprolactone (PCL) framework is loaded into a syringe, and heated to approximately 80°C to melt a polymer. The above-described pre-gel type of three-dimensional printing composition is loaded into another syringe, and the temperature is maintained at approximately below 15°C, preferably approximately 4 to 10°C. Pneumatic pressure is applied in the range of 400 to 650 kPa for fabrication of the PCL framework. The pre-gel form of composition is sprayed using a plunger-based low-dosage dispensing system.

In addition, the printing using the first bioprinting composition and the second bioprinting composition alternately may be conducted by spraying only the pre-gel form of composition using a plunger-based low-dosage dispensing system without the polycaprolactone framework.

In one embodiment, laminates using the first bioprinting composition and the second bioprinting composition alternately may laminate the first bioprinting composition and the second bioprinting composition so as to have a certain crossing angle with each other.

In addition, the first bioprinting composition and the second bioprinting composition may be printed in a form of fibers, tapes, or fabrics.

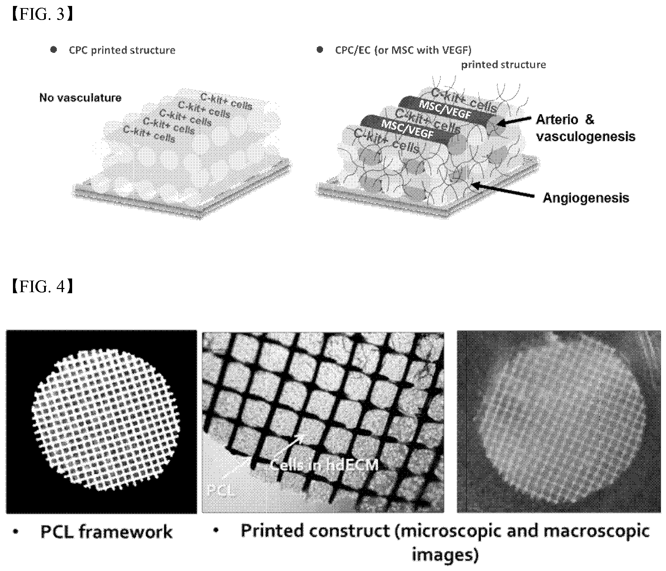

As one example, the first bioprinting composition and the second bioprinting composition may be printed on one layer in the form of fibers respectively, and the one layer may be a layer which is prepared in the form of unidirectional fibers in which the first bioprinting composition-printed fiber and the second bioprinting composition-printed fiber are alternated each other, and another layer may be laminated so as to have a certain crossing angle with the layer. That is, it may be a structure laminated by stacking 0-degree direction 1 layer and 90-degree direction 1 layer, and one example of this laminated form is shown in FIG. 3.

In one embodiment, the crosslinking agent may be riboflavin. The riboflavin may be contained in an amount of 0.001 to 0.1 % by weight, preferably 0.01 to 0.1 % by weight, based on the first bioprinting composition or the second bioprinting composition. The reason for establishing the range is because there is an effect of showing similar mechanical properties to cardiac tissues.

In addition, the respective layer-by-layer process may be conducted through printing and crosslinking under UVA. By repeatedly conducting the printing and crosslinking under UVA, that is conducting a layer-by-layer process, a three-dimensional construct shape is formed.

There is an effect of preparing a three-dimensional construct having uniformly high mechanical strength by thermal gelating the obtained three-dimensional construct shape, after constructing a three-dimensional construct shape by conducting a layer-by-layer process through a three-dimensional printing and crosslinking under UVA using riboflavin as a crosslinking agent.

The crosslinking under UVA may use 315 to 400 nm, 325 to 390 nm, 335 to 380 nm, 345 to 370 nm, 355 to 365 nm, preferably approximately 360 nm wavelength of UVA.

In addition, the crosslinking under UVA may be conducted for 1 to 10 min, 1 to 9 min, 1 to 8 min, 1 to 7 min, 1 to 6 min, 1 to 5 min, 1 to 4 min, 2 to 10 min, 2 to 9 min, 2 to 8 min, 2 to 7 min, 2 to 6 min, 2 to 5 min, 2 to 4 min, preferably approximately 3 min.

That is, it is revealed that a three-dimensional construct having uniformly high mechanical strength can be prepared by a crosslinking-thermal gelation including a use of riboflavin by the present invention. Thus, not only high mechanical strength can be provided by the crosslinking-thermal gelation using riboflavin, but also a problem of post-print crosslinking which requires a use of toxic crosslinking agents such as glutaraldehyde and causes heterogeneous crosslinking in the three-dimensional construct can be avoided.

Thus, the three-dimensional construct may have a modulus at 1 rad/s of 1 to 100 kPa, 1 to 90 kPa, 1 to 80 kPa, 1 to 70 kPa, 1 to 60 kPa, 1 to 50 kPa, 1 to 40 kPa, 1 to 30 kPa, 1 to 20 kPa, for example, 10.58 kPa. The reason for establishing the range is because there is an effect of stimulating surrounding environment with similar mechanical strength to cardiac muscle.

In one embodiment, the first bioprinting composition and the second bioprinting composition independently, may further comprise, based on the total weight of each composition, 0.001 to 0.1 % by weight of riboflavin; 0.03 to 30 % by weight of one or more kinds of acids selected from the group consisting of acetic acid and hydrochloric acid; 0.1 to 0.4 % by weight of one or more kinds of proteases selected from the group consisting of pepsin and matrix metalloproteinase (MMP); and a pH control agent.

The first bioprinting composition and the second bioprinting composition prepared by the above embodiment,
may be prepared by a preparation method comprising;
a step of adding decellularized extracellular matrix to a solution of one or more kinds of acids selected from the group consisting of acetic acid and hydrochloric acid;
a step of adding one or more kinds of proteases selected from the group consisting of pepsin and matrix metalloproteinase; and
a step of adding riboflavin, pH control agent and cardiac progenitor cells (in case of the first bioprinting composition), or riboflavin, pH control agent, mesenchymal stem cells and vascular endothelial growth factor (in case of the second bioprinting composition).

The step of obtaining a solution in which the protease is added may conduct stirring after adding the protease, and the stirring may be carried out until a complete dissolution of decellularized extracellular matrix is achieved, and for example, it may be carried out typically for 24 to 48 hrs, but not limited thereto.

The printing and crosslinking may be conducted at below 15°C to prevent gelation, and for example, it is preferable that it is conducted at a low temperature of 4 to 15°C, preferably 4 to 10°C.

In addition, the thermal gelation may be carried out over 15°C, and for example, it may be conducted at a temperature of 20°C and higher, 25°C and higher, preferably, 20°C to 50°C, 20 °C to 40 °C, 20 °C to 37 °C, 25°C to 50°C, 25°C to 40°C, more preferably, 25°C to 37°C.

The first bioprinting composition and the second bioprinting composition prepared by the preparation method is a form of pH-adjusted pre-gel, and it is preferable to refrigerate them at approximately 4°C.

In addition, the thermal gelation may be carried out over 15°C, and for example, it may be conducted at a temperature of 20°C and higher, 25°C and higher, preferably, 20°C to 50°C, 20 °C to 40 °C, 20 °C to 37°C, 25°C to 50 °C, 25°C to 40°C, more preferably, 25°C to 37°C. The reason for establishing the range is because there is an effect of physically crosslinking decellularized extracellular matrix. The thermal gelation can be conducted in an incubator (humid incubator).

Moreover, the thermal gelation may be carried out for 5 to 60 min, 10 to 50 min, 15 to 40 min, preferably 20 to 30 min. The reason for establishing the range is because it is a range required to causing physical crosslinking reaction.

The thickness of the three-dimensional construct prepared by the preparation method may be controlled depending on the amount of cells to be transferred, and it may have a thickness of 50 to 1000 *µ*m, 50 to 900 *µ*m, 50 to 800 *µ*m, 50 to 700 *µ*m, 50 to 600 *µ*m, 50 to 500 *µ*m, 100 to 1000 *µ*m, 100 to 900 *µ*m, 100 to 800 *µ*m, 100 to 700 *µ*m, 100 to 600 *µ*m, 100 to 500 *µ*m, 200 to 1000 *µ*m, 200 to 900 *µ*m, 200 to 800 *µ*m, 200 to 700 *µ*m, 200 to 600 *µ*m, 200 to 500 *µ*m, 300 to 1000 *µ*m, 300 to 900 *µ*m, 300 to 800 *µ*m, 300 to 700 *µ*m, 300 to 600 *µ*m, 300 to 500 *µ*m, 400 to 1000 *µ*m, 400 to 900 *µ*m, 400 to 800 *µ*m, 400 to 700 *µ*m, 400 to 600 *µ*m, 400 to 500 *µ*m, for example a thickness of 500 *µ*m.

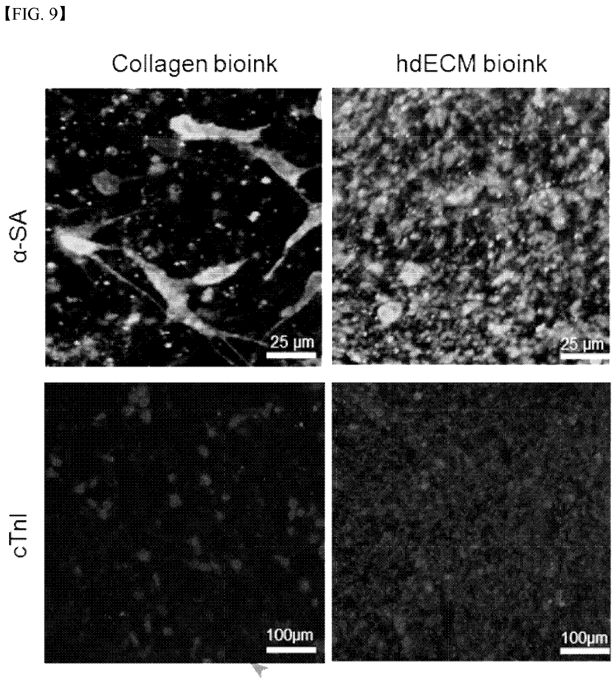

The preparation method not only equally positions the cardiac progenitor cells in the construct but also implements a vascular network composed of vascular cells in the construct, so that the viability of cells can be maintained for a long time and the cell transfer efficiency into the myocardium can be significantly improved, and the figure that the cardiac progenitor cells are equally positioned in the construct is shown in FIG. 9.

Another example of the present invention relates to a three-dimensional construct for regenerating a cardiac muscle tissue.

The three-dimensional construct for regenerating a cardiac muscle tissue may comprise one or more structures in which the first bioprinting composition containing a tissue engineering construct forming solution containing decellularized extracellular matrix and a crosslinking agent, and cardiac progenitor cells, and the second bioprinting composition containing the tissue engineering construct forming solution, mesenchymal stem cells and a vascular endothelial growth factor are alternately laminated by printing and crosslinking.

The matters relating to the first bioprinting composition, the second bioprinting composition, decellularized extracellular matrix, cardiac progenitor cells, and vascular endothelial growth factor are the same as described above.

The structure in which the first bioprinting composition and the second bioprinting composition are alternately laminated by printing and crosslinking, may be a structure in which the first bioprinting composition and the second bioprinting composition are laminated so as to have a certain crossing angle with each other. In addition, the first bioprinting composition and the second bioprinting composition may be printed in a form of fibers, tapes, or fabrics.

As one example, the first bioprinting composition and the second bioprinting composition may be printed on one layer in the form of fibers respectively, and the one layer may be a layer which is prepared in the form of unidirectional fibers in which the first bioprinting composition-printed fiber and the second bioprinting composition-printed fiber are alternated each other, and another layer may be laminated so as to have a certain crossing angle with the layer. That is, it may be a structure laminated by stacking 0-degree direction 1 layer and 90-degree direction 1 layer. The structure in which each layer is laminated once may have a thickness of approximately 50 *µ*m.

A thickness of the three-dimensional construct may be controlled depending on the amount of cells to be transferred, and it can have a thickness of 50 to 1000 *µ*m, 50 to 900 *µ*m, 50 to 800 *µ*m, 50 to 700 *µ*m, 50 to 600 *µ*m, 50 to 500 *µ*m, 100 to 1000 *µ*m, 100 to 900 *µ*m, 100 to 800 *µ*m, 100 to 700 *µ*m, 100 to 600 *µ*m, 100 to 500 *µ*m, 200 to 1000 *µ*m, 200 to 900 *µ*m, 200 to 800 *µ*m, 200 to 700 *µ*m, 200 to 600 *µ*m, 200 to 500 *µ*m, 300 to 1000 *µ*m, 300 to 900 *µ*m, 300 to 800 *µ*m, 300 to 700 *µ*m, 300 to 600 *µ*m, 300 to 500 *µ*m, 400 to 1000 *µ*m, 400 to 900 *µ*m, 400 to 800 *µ*m, 400 to 700 *µ*m, 400 to 600 *µ*m, 400 to 500 *µ*m, for example, a thickness of 500 *µ*m.

### [Effect of the Invention]

The present invention performs a three-dimensional printing using a bioprinting composition(s) comprising certain cells, in that cardiac progenitor cells and/or mesenchymal stem cells, and vascular endothelial growth factor (VEGF), thereby effectively implementing micro-environment of the cardiac muscle tissue.

In addition, the preparation method of the present invention not only equally positions the cardiac progenitor cells in the construct but also implements a vascular network composed of vascular cells in the construct, so that the viability of cells can be maintained for a long time and the cell transfer efficiency into the myocardium can be significantly improved. Therefore, the preparation method of the present invention can be usefully applied to preparation of a three-dimensional construct for treating various heart diseases including ischemic heart diseases.

### [Brief Description of Drawings]

FIG. 1 is a photograph of the decellularized extracellular matrix (hdECM) obtained from a cardiac tissue according to one example of the present invention.
FIG. 2 is an optical microscope photograph and a tissue staining photograph of the decellularized extracellular matrix (hdECM) obtained from a cardiac tissue according to one example of the present invention
FIG. 3 is a mimetic diagram of the three-dimensional construct prepared according to one example of the present invention.
FIG. 4 is photographs showing a shape of the prepared three-dimensional construct using PCL framework according to one example of the present invention.
FIG. 5 is an echocardiography m-mode photograph taken before and 8 weeks after construct transplantation, after transplanting the three-dimensional construct prepared according to one example of the present invention into a rat myocardial infarction model.
FIG. 6 is a photography showing cardiac function measured before, 4 weeks and 8 weeks after construct transplantation, after transplanting the three-dimensional construct prepared according to one example of the present invention into a rat myocardial infarction model, in numerical form.
FIG. 7 is the result of performing a histological analysis by sacrificing after 8 weeks, after transplanting the three-dimensional construct prepared according to one example of the present invention, and is a photograph observing the degree of fibrosis of the myocardial tissue and the change of myocardial wall thickness by performing masson's trichrome staining.
FIG. 8 is a photograph showing the regeneration degree of functional myocardium and the regeneration degree of blood vessel of the myocardial infarction region by immunofluorescence staining according to one example of the present invention.
FIG. 9 is a photograph showing cardiac progenitor cells uniformly positioned in the three-dimensional construct for regenerating a cardiac muscle tissue prepared according to one example of the present invention.

### [Detailed Description of the Embodiments]

Hereinafter, the present invention will be described in more detail by the following examples. However, the examples are given for illustrating the present invention only, and the scope of the present invention is not limited by the examples.

### Preparation Example 1: Preparation of the first bioprinting composition

### 1-1: Preparation of decellularized extracellular matrix

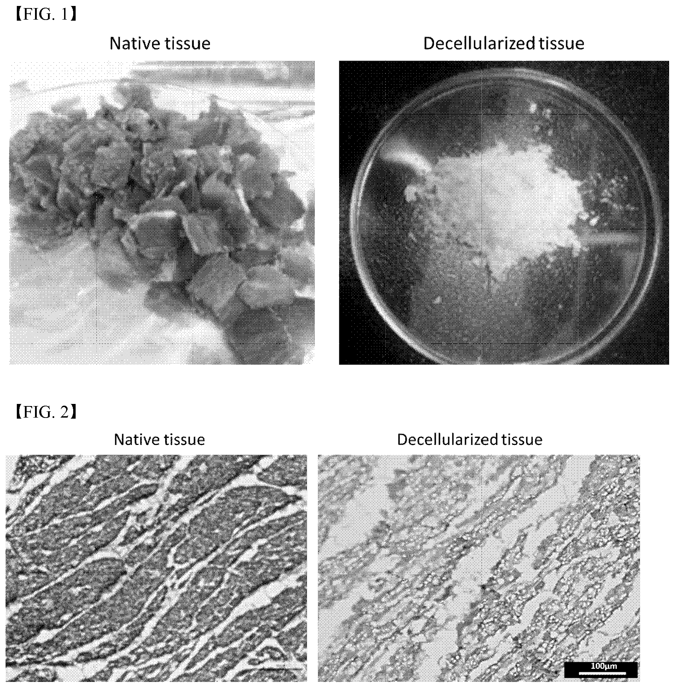

Decellularized extracellular matrix was prepared according to a method disclosed in Falguni Pati, et al., Nat Commun. 5, 3935 (2014) using porcine cardiac tissues (hereinafter, 'hdECM'). The prepared hdECM was finally lyophilized and kept frozen before used. The optical microscope photograph and tissue staining photograph were shown in FIG. 2.

### 1-2: Preparation of pre-gel form of a tissue engineering construct forming solution

Liquid nitrogen was poured to the obtained lyophilized hdECM and it was crushed with mortar and a pestle. After the obtained hdECM powder (330 mg) was added to 0.5 mol/l acetic acid aqueous solution (10ml), and pepsin (33mg) (P7125, Sigma-Aldrich) was added, it was stirred for 48 hrs at a room temperature. Maintaining the temperature of the obtained solution below 10°C, riboflavin (2mg) was added and 10 mol/l NaOH solution which was cooled below 10°C was added, thereby controlling pH as approximately pH 7. The obtained pre-gel form of a tissue engineering construct forming solution was refrigerated at approximately 4°C.

### 1-3: Preparation of a printing composition containing pre-gel form of a tissue engineering construct forming solution and cells

Just before conducting a printing process, 5 X 10⁶ cells/ml of cardiac progenitor cells were added to the pre-gel form of the tissue engineering construct forming solution, thereby preparing the first bioprinting composition. The cardiac progenitor cells were obtained from human cardiac muscle tissue-derived cardiac progenitor cells, Pusan National University School of Medicine, Department of Physiology.

### Preparation Example 2: Preparation of the second bioprinting composition

### 2-1: Preparation of decellularized extracellular matrix

Decellularized extracellular matrix was prepared according to a method disclosed in Falguni Pati, et al., Nat Commun. 5, 3935 (2014) using porcine cardiac tissues (hereinafter, 'hdECM'). The prepared hdECM was finally lyophilized and kept frozen before used. The optical microscope photograph and tissue staining photograph were shown in FIG. 2.

### 2-2: Preparation of pre-gel form of a tissue engineering construct forming solution

Liquid nitrogen was poured to the obtained lyophilized hdECM and it was crushed with mortar and a pestle. After the obtained hdECM powder (330 mg) was added to 0.5 mol/l acetic acid aqueous solution (10ml), and pepsin (33mg) (P7125, Sigma-Aldrich) was added, it was stirred for 48 hrs at a room temperature. Maintaining the temperature of the obtained solution below 10°C, riboflavin (2mg) was added and 10 mol/l NaOH solution which was cooled below 10°C was added, thereby controlling pH as approximately pH 7. The obtained pre-gel form of a tissue engineering construct forming solution was refrigerated at approximately 4°C.

### 2-3: Preparation of a printing composition containing pre-gel form of a tissue engineering construct forming solution and cells

Just before conducting a printing process, 5 X 10⁶ cells/ml of mesenchymal stem cells and vascular endothelial growth factor (serial number: 293-VE-10, R&D systems company) (100 ng/ml) were added respectively to the pre-gel form of the tissue engineering construct forming solution, thereby preparing the second bioprinting composition. The mesenchymal stem cells were inferior turbinate-derived mesenchymal stem cells obtained from Catholic University of Korea, Department of Otolaryngology Medical Science.

### Comparative Example 1: Preparation of a three-dimensional construct for tissue engineering

A three-dimensional construct was fabricated according to the method disclosed in Falguni Pati, et al., Nat Commun. 5, 3935 (2014) using the first bioprinting composition obtained from the preparation example 1.

Specifically, polycaprolactone (PCL) framework was loaded on the syringe (the first syringe) of multi-head tissue and organ printing system (Jin-Hyung Shim et al., J. Micromech. Microeng. 22 085014 (2012)), and it was heated at approximately 80°C to melt a polymer. The pre-gel form of first bioprinting composition obtained from the preparation example 1 was loaded on another syringe (the second syringe), and the temperature was maintained below approximately 10°C. A thin PCL framework having below approximately 100 um line width, approximately 300 um of gap and 120 um of thickness by putting approximately 600 kPa of pneumatic pressure to the first syringe, and after spraying the contents of the second syringe on the PCL framework, approximately 360 nm of UVA was radiated for 3 min, thereby crosslinking. Then, a layer-by-layer process through spraying the contents of the second syringe and the crosslinking was conducted, thereby forming a three-dimensional construct shape. The obtained three-dimensional construct shape was thermally gelated by putting into approximately 37°C of incubator (humid incubator) and maintaining for 30 min, thereby preparing a three-dimensional construct (refer to 'CPC printed'). The obtained three-dimensional construct has approximately 300 to 400 um thickness.

### Example 1: Preparation of a three-dimensional construct for tissue engineering

A three-dimensional construct was fabricated using the first bioprinting composition and the second bioprinting composition obtained from the preparation examples 1 and 2.

Specifically, polycaprolactone (PCL) framework was loaded on the syringe (the first syringe) of multi-head tissue and organ printing system (Jin-Hyung Shim et al., J. Micromech. Microeng. 22 085014 (2012)), and it was heated at approximately 80°C to melt a polymer. The pre-gel form of the first bioprinting composition obtained from the preparation example 1 and the second bioprinting composition obtained from the preparation example 2 were loaded on another syringe (the second and third syringes, respectively), and the temperature was maintained below approximately 10°C. A thin PCL framework having below approximately 100 um line width, approximately 300 um of gap and 120 um of thickness by putting approximately 600 kPa of pneumatic pressure to the first syringe, and after spraying the contents of the second syringe on the PCL framework, approximately 360 nm of UVA was radiated for 3 min, thereby crosslinking. Then, a layer-by-layer process through spraying the contents of the second and third syringes and the crosslinking was conducted, thereby forming a three-dimensional construct shape.

The obtained three-dimensional construct shape was thermally gelated by putting into approximately 37°C of incubator (humid incubator) and maintaining for 30 min, thereby preparing a three-dimensional construct for tissue engineering (refer to 'CPC/MSC printed'). The obtained three-dimensional construct has approximately 300 to 400 um thickness, and its shape was same as FIG. 3.

### Test Example 1. Measurement of complex modulus with or without riboflavin addition

After crosslinking the pre-gel form of solution obtained from the preparation example 1 by irradiating approximately 360 nm of UVA for 3 min, it was put into approximately 37°C of incubator (humid incubator) and maintained for 30 min, thereby inducing a thermal gelation and forming a hydrogel (crosslinked hydrogel A). In addition, a pre-gel form of a tissue engineering construct forming solution prepared according to the same method as the preparation example 1 except using no riboflavin was put into approximately 37°C of incubator (humid incubator) and maintained for 30 min, thereby inducing a thermal gelation and forming a hydrogel (hydrogel B).

Then, as to the obtained respective hydrogel, complex modulus at 1 rad/s frequency was measured and the result was as the following Table 1.

**[Table 1]**

| | |
|---|---|
| classification | modulus (n=3, 1 rad/s) |
| crosslinked hydrogel A | 10.58 ± 3.4 kPa |
| hydorgel B | 0.33 ± 0.13 kPa |

As can be seen from the result of Table 1, the crosslinked hydrogel A according to the present invention has 10.58 kPa modulus at 1 rad/s, and it was demonstrated that it has approximately over 30-fold strength enhancement by crosslinking than the hydrogel B where only gelation treatment was carried out without crosslinking.

### Test Example 2.

The chest of 4 to 5-week-old rats (weight: 300±30g) was incised and permanent ligation of Left Anterior Descending artery (LAD) was performed, thereby inducing myocardial infarction.

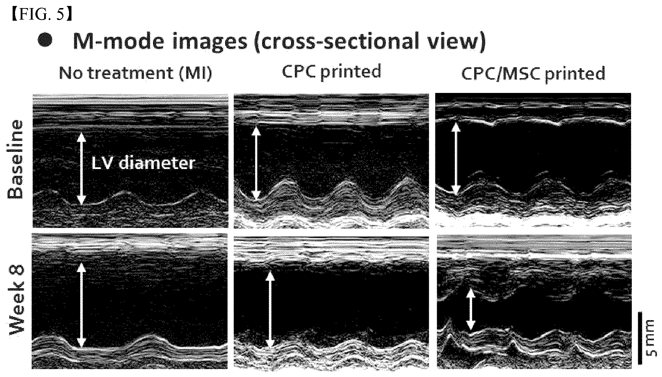

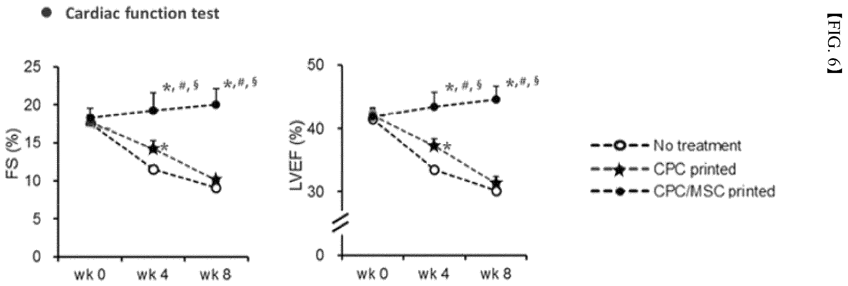

Seven days later, the three-dimensional construct prepared in the comparative example 1 (CPC printed) and the three-dimensional construct prepared in the example 1 (CPC/MSC printed) were cut into 8 mm diameters, respectively, and after transplanted into myocardial infarction region, sutured (respectively, n=7). The result of confirming left ventricle diameter (LV diameter) by echocardiography at 4 and 8 weeks after transplantation was shown in FIG. 5, and the result of performing significance evaluation between experimental samples by the student T-test by calculating left ventricle ejection fraction (LVEF) and fractional shortening (FS) which show myocardial functions was shown in FIG. 6.

As can be seen in FIGs. 5 and 6, the left ventricular internal diameter was significantly reduced compared with the experimental group (control group) which had not been subjected to any treatment after 4 and 8 weeks after transplantation of the three-dimensional construct according to example 1. In addition, it can be observed that the left ventricular systolic function was significantly increased in the CPC/MSC group compared to the CPC group, and this shows that a cardiac remodeling was inhibited by the construct.

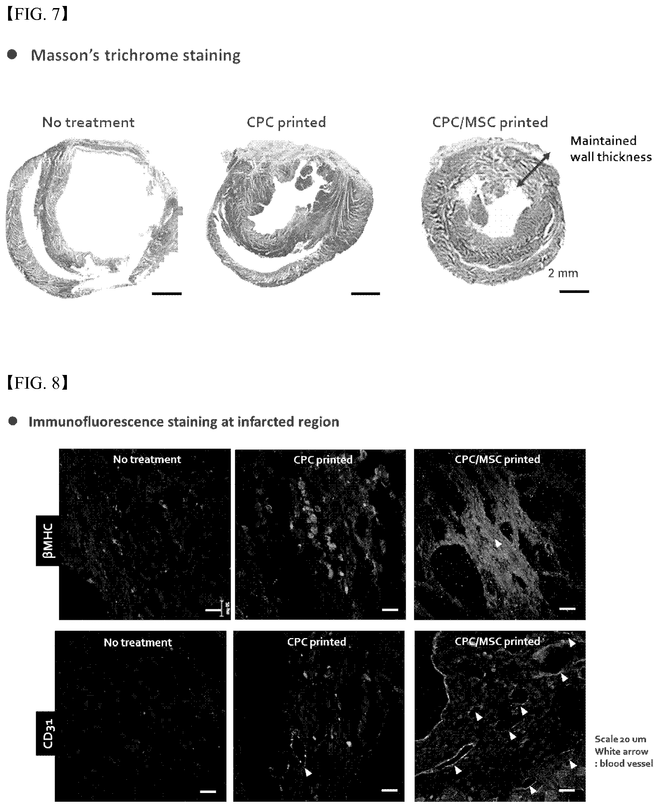

After harvesting the heart of rats at 8 weeks after transplantation and collecting tissues at the same location (approximately 1/3 point from the apex of the heart in the base direction) per object (3um thickness), Masson's trichrome staining was carried out. The heart muscle (red) and the scar tissue of the myocardial infarction region (blue) were discriminated using the staining method, and the result was shown in FIG. 7.

As can be seen from FIG. 7, the thickness of the inner wall of the left ventricle after 8 weeks of the three-dimensional construct transplantation prepared according to the present invention was kept thicker than the control group, and the formation of scar tissues of the myocardial infarction region was less. In addition, in contrast to the three-dimensional construct formed by only the first bioprinting composition (CPC printed) as in the comparative example 1, when the three-dimensional construct formed by the first bioprinting composition and the second bioprinting composition (CPC/MSC printed) as the example 1 was transplanted, the formation of scar tissues was more effectively inhibited, and the thickness of the inner wall of the left ventricle was not thinned and was maintained.

As can be seen from FIG. 8, the expression of β-myosin heavy chain (βMHC) representing contractibility of cardiac muscle tissue and cluster of differentiation 31 (CD31) representing angiogenesis on the myocardial infarction region at which the three-dimensional construct prepared according to the present invention was transplanted was significantly increased, and in particular, when the three-dimensional construct (CPC/MSC printed) (example 1) formed by the first bioprinting composition and the second bioprinting composition was transplanted, the effect was more maximized.

Thus, it was demonstrated that the three-dimensional construct prepared by the preparation method of the present invention can maintain the viability of cells for a long time by implementing a vascular network in the construct (that is, by implementing a microenvironment of the cardiac muscle tissue effectively), thereby significantly improving the cell transfer efficiency into the myocardium.

## Claims

1. A method of preparing a three-dimensional construct for regenerating a cardiac muscle tissue comprising,
(a) forming a three-dimensional construct by printing and crosslinking a first bioprinting composition comprising a tissue engineering construct forming solution containing decellularized extracellular matrix and a crosslinking agent, and cardiac progenitor cells, and a second bioprinting composition comprising the tissue engineering construct forming solution, mesenchymal stem cells and a vascular endothelial growth factor, to arrange the first bioprint layer and the second bioprint layer alternately; and
(b) carrying out the thermal gelation for three-dimensional construct by thermally gelating the crosslinked three-dimensional construct,
wherein the (a) step is performed at a temperature at which no thermal gelation occurs, and the (b) step is performed at a temperature at which thermal gelation occurs.

2. The preparation method of claim 1, wherein the decellularized extracellular matrix is derived from a cardiac tissue.

3. The preparation method of claim 1, wherein the decellularized extracellular matrix of the first bioprinting composition is contained in an amount of 1 to 4 % by weight based on the total weight of the first bioprinting composition.

4. The preparation method of claim 1, wherein the decellularized extracellular matrix of the second bioprinting composition is contained in an amount of 1 to 4 % by weight based on the total weight of the second bioprinting composition.

5. The preparation method of claim 1, wherein the cardiac progenitor cells are contained in a range of 10⁵ to 10⁸ cells/ml in the first bioprinting composition.

6. The preparation method of claim 1, wherein the mesenchymal stem cells are contained in a range of 10⁵ to 10⁸ cells/ml in the second bioprinting composition.

7. The preparation method of claim 1, wherein the vascular endothelial growth factor is contained in a range of 50 to 1000ng/ml in the second bioprinting composition.

8. The preparation method of claim 1, wherein the tissue engineering construct forming solution has a pH of 6.5 to 7.5, and further comprises an acid and a protease.

9. The preparation method of claim 8, wherein the tissue engineering construct forming solution comprises 0.03 to 30 % by weight of acid; and 0.1 to 0.4 % by weight of protease, based on the total weight of the bioprinting composition.

10. The preparation method of claim 8, wherein the tissue engineering construct forming solution, further comprises;
one or more kinds of acids selected from the group consisting of acetic acid and hydrochloric acid;
one or more kinds of proteases selected from the group consisting of pepsin and matrix metalloproteinase (MMP); and
a pH control agent.

11. The preparation method of claim 1, wherein the first bioprinting composition and the second bioprinting composition independently, further comprise,
one or more kinds of cells selected from the group consisting of endothelial progenitor cells, endothelial cells and cardiomyocytes; and
one or more kinds of growth factors selected from the growth factor group consisting of fibroblast growth factor (FGF), platelet-derived growth factor (PDGF), angiopoietin-1, transforming growth factor beta (TGF-β), erythropoietin (EPO), stem cell factor (SCF), epidermal growth factor (EGF) and colony stimulating factor (CSF).

12. The preparation method of claim 11, wherein the first bioprinting composition and the second bioprinting composition independently, further comprise, one or more kinds of enzymes selected from the enzyme group consisting of matrix metalloproteinase (MMP) and tissue inhibitor matrix metalloproteinase (TIMP).

13. The preparation method of claim 1, wherein the first bioprinting composition and the second bioprinting composition independently, have a viscosity at a shear rate of 1 s⁻¹ measured at 15°C in the range of 1 to 30 Pa·S.

14. The preparation method of claim 1, wherein the crosslinking agent is riboflavin.

15. The preparation method of claim 1, wherein the crosslinking agent is comprised independently in the first bioprinting composition and the second bioprinting composition in an amount of 0.001 to 0.1 % by weight, based on the total weight of each composition.

16. The preparation method of claim 1, wherein the printing and crosslinking are performed below 15°C.

17. The preparation method of claim 1, wherein the crosslinking is performed for 1 to 10 minutes.

18. The preparation method of claim 1, wherein the thermal gelation is performed at 25°C to 37°C.

19. The preparation method of claim 1, wherein the three-dimensional construct for regenerating a cardiac muscle tissue has a thickness of 50 to 1000 *µ*m.

20. A three-dimensional construct for regenerating a cardiac muscle tissue,
wherein the three-dimensional construct for regenerating a cardiac muscle tissue is formed by performing crosslinking and thermal gelation a first bioprint layer and a second bioprint layer which are prepared by printing the first bioprinting composition and the second bioprinting composition,
wherein the first bioprinting composition comprises a tissue engineering construct forming solution containing decellularized extracellular matrix and a crosslinking agent, and cardiac progenitor cells,
wherein the second bioprinting composition comprises the tissue engineering construct forming solution, mesenchymal stem cells and a vascular endothelial growth factor.

21. The three-dimensional construct for regenerating a cardiac muscle tissue of claim 20, wherein the first bioprint layer, the second bioprint layer or both are in a form of fibers, tapes, or fabrics.

22. The three-dimensional construct for regenerating a cardiac muscle tissue of claim 20, wherein the first bioprint layer and the second bioprint layer are independently in a form of unidirectional fibers, and the bioprint layer and the second bioprint layer are laminated so as to have a crossing angle with each other.

23. The three-dimensional construct for regenerating a cardiac muscle tissue of claim 20, wherein the three-dimensional construct for regenerating a cardiac muscle tissue has a thickness of 50 to 1000 *µ*m.

24. The three-dimensional construct for regenerating a cardiac muscle tissue of claim 20, wherein the three-dimensional construct for regenerating a cardiac muscle tissue has a modulus of 1 to 100kPa at 1 rad/s.
